Europäisches Patentamt

European Patent Office

Office européen des brevets

(19)

(11) Numéro de publication : **0 022 724**
**B1**

# (12) FASCICULE DE BREVET EUROPÉEN

(45) Date de publication du fascicule du brevet :
**30.01.85**

(21) Numéro de dépôt : **80401055.1**

(22) Date de dépôt : **11.07.80**

(51) Int. Cl.⁴ : **A 61 F   2/00**, A 61 L 17/00

(54) **Implant biodégradable utilisable comme pièce de prothèse osseuse.**

(30) Priorité : **12.07.79 FR 7918120**

(43) Date de publication de la demande :
**21.01.81 Bulletin 81/03**

(45) Mention de la délivrance du brevet :
**30.01.85 Bulletin 85/05**

(84) Etats contractants désignés :
**AT BE CH DE GB IT LI LU NL SE**

(56) Documents cités :
**DE-A- 2 652 611**
**DE-A- 2 717 506**
**DE-A- 2 724 972**
**DE-A- 2 725 665**
**FR-A- 2 223 325**
**FR-A- 2 243 915**
**FR-A- 2 283 104**
**FR-A- 2 301 488**
**FR-A- 2 361 437**
**FR-A- 2 413 343**
**GB-A- 1 487 181**
**US-A- 3 787 900**
**US-A- 3 890 107**
**US-A- 3 918 100**
**US-A- 3 919 723**
**US-A- 4 149 894**
**BIOMEDIZINISCHE TECHNIK, vol. 23, no. 5, mai 1978,
N 507, pages 118-119**
**PHILIPS TECHN. REVUE, vol. 37, no. 9/10, 1977 J G J
PEELEN et al.: "Sintered hydroxylapatite as a bioceramic, pages 234-236**
**NATURE, vol. 247, 25 janvier 1974, pages 220-222**

(73) Titulaire : **ANVAR Agence Nationale de Valorisation
de la Recherche
43, rue Caumartin
F-75436 Paris Cédex 09 (FR)**

(72) Inventeur : **Guillemin, Geneviève
7, rue des Acacias
F-75017 Paris (FR)**
Inventeur : **Patat, Jean-Louis
8, rue Muller
F-75018 Paris (FR)**
Inventeur : **Patel, Alain
6, avenue Adrien Hébrard
F-75016 Paris (FR)**

(74) Mandataire : **Nony, Michel
Cabinet Nony 29, rue Cambacérès
F-75008 Paris (FR)**

## Description

La présente invention a pour objet un implant biodégradable utilisable comme pièce de prothèse osseuse.

L'invention a plus particulièrement pour objet un implant biodégradable pouvant servir de guide à la repousse osseuse et permettant notamment de combler des pertes de substances osseuses ou de remplacer des tronçons diaphysaires après résection.

La présente invention a également pour but de faciliter l'ancrage des endoprothèses articulaires.

On a déjà proposé pour la réparation ou le remplacement d'os brisés ou d'articulations endommagées, la mise en place d'implants prothétiques en matériau synthétique biocompatible avant des propriétés mécaniques acceptables.

Les difficultés rencontrées avec les procédés de fixation des pièces d'ostéosynthèse par vissage ou enclouage ont conduit à l'utilisation de ciments acryliques, comme le méthacrylate de méthyle.

Toutefois, en raison de l'utilisation de la polymérisation exothermique in situ entraînant une nécrose osseuse à l'interface, et en raison de la toxicité générale du monomère, la fixation obtenue demeure aléatoire à long terme.

C'est pourquoi la recherche s'est orientée vers de nouvelles méthodes de fixation d'une prothèse à l'os vivant.

Il existe actuellement deux voies de recherche, la première étant l'établissement de liens chimiques intimes entre l'implant et l'os, la deuxième étant l'ancrage biologique par pousse osseuse dans des prothèses réalisées en matériau poreux.

La large surface d'os néo-formé devrait permettre, avec ces méthodes, une bonne distribution des contraintes mécaniques, et éviter la résorption osseuse par hyperpression localisée.

En raison de l'importance des contraintes mécaniques auxquelles sont soumises les articulations portantes, comme la hanche ou le genou, l'utilisation de matériaux très résistants est nécessaire.

C'est ainsi que l'on a étudié et même utilisé chez l'homme des métaux poreux ou des céramiques poreuses.

Toutefois, la difficulté du réglage de la dimension des pores et de l'établissement de canaux de liaison entre pores voisins, est la source de grandes difficultés techniques pour l'obtention de tels matériaux poreux synthétiques.

Il a également été proposé dans le brevet français n° 2,223.325 d'utiliser pour la confection de prothèses ou d'implants osseux une matière synthétique constituée par de l'hydroxyapatite ou de la whitlockite ayant une microstructure correspondant à celle de la matière du squelette poreux de certains organismes marins, notamment d'échinodermes ou de coraux édificateurs de récifs.

Dans ce brevet français il est indiqué que l'emploi de matériau à base de ces squelettes naturels eux-mêmes directement pour la réalisation d'implants, de substituts osseux ou d'autres éléments de prothèse, présenterait divers inconvénients, dus notamment à la faible résistance et la solubilité élevée des carbonates naturels comme la calcite et l'aragonite qui forment la majeure partie desdits matériaux de squelettes marins.

On pensait en outre que le matériau prothétique devait avoir autant que possible une composition voisine de celle de l'os et contenir notamment des phosphates calciques qui semblaient devoir favoriser la repousse osseuse.

C'est pourquoi les auteurs du brevet français n° 74.11398 cherchaient à obtenir une structure poreuse du type hydroxyapatite. Ces matériaux poreux synthétiques sont obtenus par une réaction d'échange chimique hydrothermique nécessitant l'emploi de températures et de pressions élevées.

Il existait donc un préjugé contre l'utilisation directe des squelettes d'animaux marins invertébrés à base de carbonate de calcium.

En effet, à première vue, les matériaux à base d'hydroxyapatite semblaient présenter l'avantage d'être chimiquement et minéralogiquement très semblables aux tissus durs humains normaux et de rester stables au contact des différents liquides physiologiques alors que le carbonate ne l'est pas.

Il a maintenant été découvert, et c'est ce qui fait l'objet de la présente invention, que contrairement à ce préjugé, l'utilisation de matériau à base de carbonate de calcium présente de nombreux avantages.

C'est ainsi que la résistance et le module d'élasticité (module d'Young) de l'hydroxy-apatite et de l'aragonite de squelettes coralliens sont comparables en pratique lorsque les mesures sont effectuées à sec. Il n'en va plus de même lorsque les deux matériaux sont imprégnés de liquide, car alors les structures poreuses à base d'hydroxy-apatite deviennent très friables, ce qui constitue un inconvénient certain, pour l'utilisation envisagée, tandis que les matériaux à base de squelettes coralliens gardent leur qualité de résistance mécanique.

Il a également été découvert que la solubilité progressive des carbonates naturels dans le milieu physiologique ne constitue pas un inconvénient puisque cette résorption permet le remplacement progressif du carbonate de calcium par de l'os dur néoformé, et puisque d'autre part le carbonate de calcium est apparu, contrairement à un autre préjugé des hommes de métier, comme susceptible de favoriser davantage la repousse osseuse que les phosphates de calcium.

Au contraire, l'expérience a montré que les pores des matériaux à base d'hydroxy-apatite sont envahis par du tissu conjonctif mais non différencié en tissu osseux.

Un autre avantage de l'utilisation des carbonates est que, du fait de leur résorption progressive, et contrairement à ce qui se passe avec les matériaux non résorbables, il n'est pas nécessaire de mettre en place un matériau ayant une structure poreuse, ou ayant notamment une dimension des pores particulière. En effet, à mesure que la résorption progresse les pores se trouveront nécessairement, à un certain moment, à la dimension favorable à la repousse osseuse.

On a observé enfin que la repousse de l'os se produit sans formation d'une capsule fibreuse à l'interface. La formation d'une telle capsule fibreuse constitue un des inconvénients observés avec les pièces de prothèse classiques.

La présente invention a donc pour objet un implant ou une prothèse osseuse biodégradable constituée en un matériau cohérent à base de calcaire.

Pour que le calcaire constitue un matériau suffisamment cohérent et soit utilisable selon l'invention, il faut qu'il contienne le carbonate de calcium sous forme cristalline.

Selon l'invention, il est donc possible d'utiliser tout matériau naturel ou synthétique, non poreux ou, de préférence, poreux, répondant à cette définition.

L'invention a en particulier pour objet un implant ou prothèse osseuse biodégradable caractérisé par le fait qu'il est réalisé en un matériau cohérent constitué par du calcaire sous forme cristalline, et qui possède de préférence une structure poreuse, le diamètre des pores étant de préférence supérieur ou égal à 50 microns.

En effet, la présence d'une structure poreuse accélère la repousse osseuse.

Le matériau utilisable dans l'implant ou la prothèse osseuse de l'invention contient du carbonate de calcium sous forme d'aragonite ou de calcite.

Parmi les matériaux à base d'aragonite, on citera notamment ceux constitués par du squelette de corail madréporaire tel que le Porites, le Pocillopora ou le Favites.

Dans le squelette de Favites, les pores ont une ligne directrice et en conséquence un tel squelette pourra être utilisé notamment pour remplacer des parties diaphysaires d'os longs.

Les matériaux à base de calcite peuvent être constitués notamment par des squelettes d'échinodermes et en particulier par des épines d'oursins.

On peut utiliser par exemple les épines de l'oursin Citaris.

L'implant ou prothèse osseuse selon l'invention peut se présenter sous la forme de pièce de comblement ou de remplacement de substance osseuse, ou sous la forme de vis ou de clou.

Selon un autre mode de réalisation de l'invention le matériau calcaire tel que défini ci-dessus se trouve à l'intérieur de parties creuses ménagées dans un élément d'endoprothèse non résorbable, lesdites parties creuses étant en communication avec l'extérieur dudit élément d'endoprothèse, lesdites communications se trouvant situées dans une zone de contact avec la substance osseuse lorsque l'implant ou la prothèse est mise en place.

Le carbonate de calcium étant progressivement remplacé par de l'os néo-formé, il est ainsi possible d'obtenir un ancrage biologique de l'élément d'endoprothèse.

C'est ainsi que l'on peut réaliser une prothèse de hanche dont la queue fémorale est partiellement creuse et dont les parties creuses sont remplies du matériau calcaire tel que défini ci-dessus. Les parties creuses sont en communication avec l'extérieur de la queue fémorale ou prothèse par des perforations elles-mêmes comblées avec ledit matériau calcaire, dans la zone où la queue fémorale est en contact avec l'os après emmanchement à force dans la cavité médullaire.

Après repousse osseuse à l'intérieur de la partie creuse, la pièce de prothèse se trouve solidement ancrée.

Cet ancrage évite l'utilisation à l'interface de ciment acrylique qui constitue la source majeure et descellements observés avec de telles prothèses.

Lorsque le matériau calcaire utilisé selon l'invention est poreux, sa porosité varie généralement de 30 à 80 %.

Les implants ou prothèses de l'invention sont notamment des pièces de comblement de pertes de substance osseuse, dont les formes et dimensions définitives sont ajustées par meulage pendant le temps opératoire afin de les adapter au comblement particulier à effectuer. Les pertes de substance osseuse proviennent notamment de traumatismes ou des excisions qu'entraîne la résection de certaines tumeurs.

Les pièces selon l'invention peuvent également être des vis qui peuvent être utilisées notamment pour la fixation des implants ou prothèses de l'invention et par exemple pour la fixation des pièces de comblement définies ci-dessus lorsque la forme du comblement ne permet pas de réaliser un ancrage de la pièce de comblement. Ces vis peuvent aussi être utilisées pour la fixation l'un contre l'autre des bords de la fracture, lorsque la forme de celle-ci s'y prête.

Ces vis sont réalisées de façon classique par filetage d'une tige, constituée par exemple par une épine d'oursin.

L'utilisation de telles vis, qui sont résorbables, permet d'éviter une seconde intervention chirurgicale qui est généralement nécessaire lorsque l'on utilise des pièces d'ostéosynthèse non résorbables ; en outre, l'utilisation des vis résorbables élimine la fragilisation de l'os pendant la période de comblement des trous après exérèse des vis métalliques intra-osseuses.

Les implants ou pièces de prothèse de l'invention peuvent également être des pièces de remplacement, destinées à remplacer des résections complètes d'un tronçon diaphysaire d'os long. Généralement on cherche à adapter par meulage la forme de la pièce de remplacement à celle des berges, toutefois, la forme desdites berges peut être modifiée de façon plus favorable à la pose de la pièce de remplacement, si nécessaire, selon les techniques opératoires usuelles.

Afin de faciliter la mise en place de la pièce de remplacement, celle-ci est façonnée per-opératoirement à la meule rotative, afin de lui donner d'une part une forme s'adaptant à la résection, et de munir d'autre part l'une de ses extrémités d'un tenon destiné à pénétrer le canal médullaire et à assurer ainsi la stabilité de la pièce de remplacement.

On peut également façonner l'autre extrémité en biseau, de façon à la faire pénétrer dans le canal médullaire avant mise en place du tenon dans le canal médullaire de l'autre extrémité de la résection. L'extrémité en biseau peut être alors maintenue en place à l'intérieur du canal médullaire par l'insertion d'un coin également réalisé avec le matériau selon l'invention.

Les implants ou prothèses de la présente invention peuvent donc avoir aussi la forme de coins destinés à maintenir en place d'autres pièces de prothèse et notamment des pièces de remplacement telles que définies ci-dessus.

Les pièces de prothèse de l'invention peuvent également se présenter sous forme de prothèses articulaires creuses dont les parties creuses sont remplies du matériau calcaire tel que défini ci-dessus.

Les implants de l'invention peuvent aussi être utilisés notamment comme réserve d'ions calciques pour favoriser la repousse osseuse dans la technique dite des allongements osseux.

Lorsque le matériau utilisé pour la réalisation des implants ou prothèses de l'invention est un morceau de squelette d'animal marin, il convient de lui faire subir des traitements destinés à les nettoyer et à éliminer les matières organiques susceptibles d'avoir des effets antigéniques néfastes.

Ces opérations comprennent des lavages et le traitement avec une solution oxydante, comme par exemple une solution d'hypochlorite de sodium qui permet de détruire les matières organiques.

Finalement, les échantillons de matériau calcaire sont stérilisés à la chaleur. Ils sont alors prêts à être utilisés chirurgicalement, étant entendu qu'ils seront façonnés pendant le temps opératoire dans des conditions stériles.

La présente invention a également pour objet l'application des implants ou prothèses tels que définis ci-dessus, au comblement ou au remplacement de pertes de substance osseuse ou de résections osseuses. Cette application peut être mise en œuvre comme indiqué ci-dessus.

Les exemples suivants illustrent l'invention sans toutefois la limiter.

Exemple 1

Traitement préliminaire du matériau constitué par un morceau de corail.

Pendant une semaine, le morceau de corail brut est abondamment rincé à l'eau courante afin de le débarrasser des organismes qui l'habitent. Il est ensuite séché à l'air libre puis débité à la scie circulaire en tronçons de différentes tailles et formes selon les besoins. Ces tronçons sont radiographiés sur un film aux définitions lentes de façon à vérifier l'homogénéité et l'intégrité de la structure interne du squelette. Les échantillons sont plongés dans une solution d'hypochlorite de sodium pendant 48 heures. Ils sont à nouveau rincés dans l'eau courante pendant 48 heures.

Les échantillons sont ensuite stérilisés à la chaleur humide (120 °C, pendant 30 minutes) et sont alors prêts à être utilisés chirurgicalement.

C'est pendant le temps opératoire que l'échantillon sera façonné de manière à prendre la forme la plus exactement désirée. Ce « modelage » ou cet « ajustage » est effectué, stérilement bien entendu, à l'aide d'une meule abrasive montée sur un moteur électrique. Lorsque l'implant a acquis la forme désirée, il est mis en place. Dans le cas d'un os long, le positionnement doit se faire de préférence parallèlement à l'axe longitudinal de l'os, afin d'aider le passage de la moelle et des vaisseaux, et de favoriser et accélérer ainsi la repousse osseuse.

Exemple 2

On a réalisé trois prothèses articulaires de hanche en vitallium. La queue fémorale a la forme d'un cylindre creux, dont la surface est irrégulière et percée de trous permettant la communication avec la partie creuse qui est remplie de morceaux de squelette de corail traités comme à l'exemple 1.

Ces prothèses sont implantées dans les diaphyses fémorales de chien. Les trous de communication de la partie creuse avec l'extérieur sont destinés à mettre en correspondance ladite partie creuse avec la corticale ou la cavité médullaire.

Après prélèvement au bout de trois mois, on a observé que des ponts osseux s'étaient formés à travers les trous de communication, établissant ainsi un ancrage biologique osseux de la prothèse. Le matériau calcaire à l'intérieur de la partie creuse de la prothèse n'était pas encore complètement résorbé.

Exemple 3

Etude du comblement de pertes de substance osseuse.

Cette expérience est réalisée sur des chiens adultes.

Après exposition de la diaphyse fémorale ou cubitale, on réalise une perte partielle de substance à l'aide du ciseau frappé. Cette perte de substance a la forme d'une encoche rectangulaire de 20 mm de long et de 6 mm d'épaisseur.

Le matériau calcaire façonné à la forme désirée est inséré et maintenu en place par une plaque d'ostéosynthèse en vitallium fixée à l'aide de vis.

On a étudié, à titre de comparaison, le comportement de pièces de comblement réalisées en hydroxy-apatite poreuse selon le procédé du brevet français n° 2,223.325.

Après le temps d'observation choisi, les animaux sont sacrifiés et des prélèvements sont effectués, fixés dans une solution de formaldéhyde, déshydratés par passage dans l'alcool, puis inclus dans une résine polyester désignée sous la dénomination commerciale AMBREX, additionnée d'un catalyseur.

Une fois l'inclusion polymérisée, on tronçonne le bloc obtenu à l'aide d'une meule diamantée de tronçonnage.

Les coupes obtenues sont polies sur des disques abrasifs diamantés de grains de plus en plus fins, puis colorés ou Bleu de toluidine à 0,2 % dans une solution tampon de pH 4,2, puis rincées. Cette coloration traduit les éléments basophiles par une teinte bleue (orthochromatique), violette (métachromasie) ou rouge pourpre (métachromasie).

D'autre part, on a suivi l'évolution radiographique en prenant des clichés à intervalles réguliers.

Après deux semaines, les implants réalisés à l'aide de squelette de Porites, des travées osseuses ont rempli l'espace médullaire sous-jacent entre la corticale et l'implant.

On observe au microscope un contact extrêmement intime, pratiquement sans discontinuité, entre le corail et l'os néo-formé. Des cellules osseuses sont visibles sur le corail.

Après quatre semaines, on observe une nette résorption de l'implant qui est étroitement enserré dans du tissu osseux. Une coupe effectuée au centre de l'implant montre une continuité parfaite entre le matériau implanté et l'os de repousse au point que la délimitation entre les deux matériaux est difficile à établir.

Après huit semaines, on retrouve au centre du lieu d'implantation une petite masse de Porites encore organisée, mais considérablement diminuée, dans une masse d'os compacte qui s'organise peu à peu en os haversien.

Après dix semaines, la résorption du Porites est radiologiquement complète.

Une micro-photographie montre que du tissu osseux en cours de minéralisation a réalisé la jonction entre les deux berges des fractions.

Après vingt semaines, on observe une disparition radiologique et histologique complète du corail implanté. L'os cortical s'est complètement reformé et organisé en os haversien.

Les implants d'hydroxy-apatite ont donné lieu aux observations suivantes :

Après deux semaines, l'implant est envahi par du tissu conjonctif.

Après quatre semaines, radiologiquement, l'état de l'implant est identique à son état initial ; histologiquement le tissu conjonctif a bien pénétré les espaces libres ; la jonction hydroxy-apatite/os est irrégulière.

Après huit semaines, la partie poreuse de l'implant est entièrement envahie par du tissu osseux. Il n'y a pas de signe évident de résorption de l'implant. Il n'a pas été possible d'observer des ostéoblastes sur l'hydroxy-apatite.

**Exemple 4**

Etude du remplacement de résections osseuses.

Ces remplacements sont réalisés par résection complète d'un tronçon diaphysaire, la berge proximale étant sciée perpendiculairement à l'axe longitudinal et la berge distale étant sciée en biais ; l'implant de Porites est façonné per-opératoirement à la meule rotative, de façon à lui donner une forme s'adaptant à la résection et de le munir du côté proximal de l'implant d'un tenon destiné à pénétrer le canal médullaire afin d'assurer une plus grande stabilité au matériau de remplacement.

Le tronçon réséqué a grossièrement la forme d'un parallélépipède dont une base mesure 18 mm et l'autre 5 mm.

Le maintien en place est assuré à l'aide d'une plaque d'ostéosynthèse en vitallium fixée par des vis.

La contension post-opératoire consiste en un pansement à l'élastoplaste dans le cas d'une résection de cubitus, et en la mise sous plâtre armé dans le cas d'une résection de fémur.

Les prélèvements sont faits de la même façon qu'à l'exemple précédent.

Après seize semaines, le volume de l'implant de Porites a considérablement diminué. Il n'y a aucune évidence radiologique de nécrose osseuse. On ne trouve plus trace radiologique du tenon qui pénétrait initialement le canal médullaire et qui a été résorbé plus rapidement que le reste de l'implant.

Après seize semaines, dans le cas d'un implant d'hydroxyapatite, on ne note pas de nécrose osseuse. L'implant est envahi par un tissu conjonctif, mais non différencié en tissu osseux.

En conclusion, l'hydroxy-apatite répliqué, dans de telles pièces de remplacement, ne constitue pas un matériau favorable à une restauration osseuse satisfaisante.

Par contre, les fractions de Porites représentent un implant très progressivement soluble dont les produits de dissociation sont directement réutilisables par les cellules osseuses en formation.

On signale en outre qu'un certain nombre d'implantations de prothèses selon l'invention ont été effectuées chez l'homme et ont donné des résultats satisfaisants.

**Revendications**

1. Implant osseux ou prothèse osseuse biodégradable, caractérisé par le fait qu'il est réalisé en un matériau cohérent constitué par du calcaire sous forme cristalline.

2. Implant ou prothèse selon la revendication 1, caractérisé par le fait qu'il possède en outre une structure poreuse.

3. Implant ou prothèse selon la revendication 2, caractérisé par le fait que le diamètre des pores est supérieur ou égal à 50 microns.

4. Implant ou prothèse selon l'une quelconque des revendications précédentes, caractérisé par le fait que ledit matériau est de l'aragonite.

5. Implant ou prothèse selon la revendication 4, caractérisé par le fait que ledit matériau est constitué par du squelette de corail madréporaire.

6. Implant ou prothèse selon la revendication 5, caractérisé par le fait que ledit corail est le Porites, le Pocillopora ou le Favites.

7. Implant ou prothèse selon l'une quelconque des revendications 1 à 3, caractérisé par le fait que ledit matériau est en calcite.

8. Implant ou prothèse selon la revendication 7, caractérisé par le fait qu'il est constitué par du squelette d'échinoderme, et en particulier par des épines d'oursins.

9. Implant ou prothèse selon la revendication 8, caractérisé par le fait que l'oursin est le Citaris.

10. Implant ou prothèse selon l'une quelconque des revendications précédentes, caractérisé par le fait qu'il se présente sous la forme de pièce de comblement ou de remplacement de substance osseuse, ou sous la forme de vis ou de clou.

11. Implant ou prothèse selon l'une quelconque des revendications 1 à 9, caractérisé par le fait que ledit matériau calcaire se trouve à l'intérieur des parties creuses d'un élément d'endo prothèse non résorbable partiellement creux, les parties creuses étant en communication avec l'extérieur dudit élément, lesdites communications se trouvant situées dans une zone de contact avec la substance osseuse lorsque l'implant ou la prothèse est mis en place.

12. Implant ou prothèse selon l'une quelconque des revendications 2 à 11, caractérisé par le fait que ledit matériau calcaire a une porosité variant de 30 à 80 %.

13. Implant ou prothèse selon l'une quelconque des revendications précédentes, caractérisé par le fait qu'il constitue des pièces de comblement, des pièces de remplacement, des vis ou coins de fixation, ou des prothèses articulaires partiellement creuses dont les parties creuses sont remplies dudit matériau calcaire.

14. Application des implants ou prothèses tels que définis dans l'une quelconque des revendications précédentes, au comblement ou au remplacement de pertes de substance osseuse ou de résections osseuses.

## Claims

1. Biodegradable bony implant or prosthesis, characterised in it that it is produced in a coherent material consisting of limestone in a crystalline form.

2. Implant or prosthesis according to Claim 1, characterised in that it also possesses a porous structure.

3. Implant or prosthesis according to Claim 2, characterised in that the pore diameter is greater than or equal to 50 microns.

4. Implant or prosthesis according to any of the preceding claims, characterised in that the said material is aragonite.

5. Implant or prosthesis according to Claim 4, characterised in that the said material consists of madrepore coral skeleton.

6. Implant or prosthesis according to Claim 5, characterised in that the said coral is Porites, Pocillopora or Favites.

7. Implant or prosthesis according to any one of Claims 1 to 3, characterised in that the said material is calcite.

8. Implant or prosthesis according to Claim 7, characterised in that it consists of echinoderm skeleton, in particular sea urchin spines.

9. Implant or prosthesis according to Claim 8, characterised in that the sea urchin is Citaris.

10. Implant or prosthesis according to any one of the preceding claims, characterised in that it takes the form of a filling piece or replacement piece for bony substance, or the form of a screw or pin.

11. Implant or prosthesis according to any one of Claims 1 to 9, characterised in that the said calcareous material is present inside the hollow parts of a partially hollow, non-resorbable endoprosthetic element, the hollow parts being in communication with the outside of the said element, the said communications being situated in a zone of contact with the bony substance when the implant or prosthesis is in place.

12. Implant or prosthesis according to any one of Claims 2 to 11, characterised in that the said calcareous material has a porosity varying from 30 to 80 %.

13. Implant or prosthesis according to any one of the preceding claims, characterised in that it constitutes filling pieces, replacement pieces, screws or wedges for attachment, or partially hollow articular prostheses, the hollow parts of which are filled with the said calcareous material.

14. Application of the implants or prostheses as defined in any one of the preceding claims, to the filling or replacement of losses of bony substance or of bony resections.

## Ansprüche

1. Biodegradables Knochenimplantat oder biodegradable Knochenprothese, dadurch gekennzeichnet, daß es bzw. sie aus einem kohärenten Material gefertigt ist, das aus einem kalkhaltigen, kristallinen Material besteht.

2. Implantat oder Prothese nach Anspruch 1, dadurch gekennzeichnet, daß es bzw. sie außerdem eine poröse Struktur besitzt.

3. Implantat oder Prothese nach Anspruch 2, dadurch gekennzeichnet, daß der Porendurchmesser größer oder gleich 50 μ ist.

4. Implantat oder Prothese nach einem der vorhergehenden Ansprüche, dadurch gekennzeichnet, daß das Material Aragonit ist.

5. Implantat oder Prothese nach Anspruch 4, dadurch gekennzeichnet, daß das Material aus dem Skelett von Schwammkorallen besteht.

6. Implantat oder Prothese nach Anspruch 5, dadurch gekennzeichnet, daß es sich bei den Korallen um Porites, Pocillopora oder Favites handelt.

7. Implantat oder Prothese nach einem der Ansprüche 1 bis 3, dadurch gekennzeichnet, daß das Material Calcit ist.

8. Implantat oder Prothese nach Anspruch 7, dadurch gekennzeichnet, daß es bzw. sie aus Echinodermenskelett, und insbesondere aus Seeigelstacheln besteht.

9. Implantat oder Prothese nach Anspruch 8, dadurch gekennzeichnet, daß es sich bei dem Seeigel um den Citaris handelt.

10. Implantat oder Prothese nach einem der vorhergehenden Ansprüche, dadurch gekennzeichnet, daß es bzw. sie als stückige Füllung oder Ersatz von Knochensubstanz, oder in Form einer Schraube oder eines Nagels vorliegt.

11. Implantat oder Prothese nach einem der Ansprüche 1 bis 9, dadurch gekennzeichnet, daß sich das kalkhaltige Material im Inneren von Hohlräumen eines teilweise hohlen, nicht resorbierbaren Endoprothesenelementes befindet, wobei die Hohlräume mit der Außenseite des Elementes in Verbindung stehen, und wobei sich die Verbindungen in einer Kontaktzone mit der Knochensubstanz befinden, wenn das Implantat oder die Prothese eingesetzt ist.

12. Implantat oder Prothese nach einem der Ansprüche 2 bis 11, dadurch gekennzeichnet, daß das kalkhaltige Material eine Porosität von 30 bis 80 % aufweist.

13. Implantat oder Prothese nach einem der vorhergehenden Ansprüche, dadurch gekennzeichnet, daß es bzw. sie als Füllstück, Ersatzstück, Schraube oder Befestigungseck oder als teilweise hohle Gelenkprothese, deren Hohlräume mit dem kalkhaltigen Material gefüllt sind, vorliegt.

14. Verwendung der Implantate oder Prothesen nach einem der vorhergehenden Ansprüche zum Auffüllen oder Ersetzen verlorener Knochensubstanz oder bei Knochenresektionen.